# EUROPEAN PATENT APPLICATION

(11) **EP 1 797 907 A2**
(43) Date of publication of application: **20.06.2007**
(21) Application number: 06255798.8
(22) Date of filing: 13.11.2006
(51) Int. Cl.: A61L 24/00

(54) **Treatment of aneurysms with an inplantable polymeric, biodegradable device incorporating a MMP inhibitor**

(30) Priority: 01.12.2005 US 292235
(71) Applicant: Cordis Corporation, Miami Lakes, FL 33014 (US)
(72) Inventor: Peng, Eileen, Basking Ridge, NJ 07920 (US)
(74) Representative: Belcher, Simon James

(57) **Abstract**

The present invention is directed to the treatment of small abdominal aortic aneurysms by the implantation of a polymeric, biodegradable device incorporating an anti-aneurismal effective amount of a MMP inhibitor. The present invention is further directed to the polymeric, biodegradable implantable device produced from a biodegradable, polymeric material having an anti-aneurismal effective amount of a MMP inhibitor incorporated therein. Not only can the treatment in accordance with the present invention prevent the inception and growth of aneurysms, it can also induce regression of established aneurysms.

## Description

The present invention relates to the treatment of aneurysms, particularly aortic aneurysms. More specifically, the present invention is directed to the treatment of small abdominal aortic aneurysms ("AAA") by the implantation of a polymeric, biodegradable device incorporating a MMP inhibitor. The present invention is also directed to the implantable polymeric, biodegradable device incorporating the MMP inhibitor.

The aorta is the body's largest artery, having roughly the diameter of a garden hose, and is the blood vessel that carries oxygen-rich blood away from the heart. The aorta extends from the heart down through the chest and the abdominal region, dividing into two smaller blood vessels that provide blood to the pelvis and legs. An aortic aneurysm is an abnormal bulge that can occur anywhere along the wall of the aorta. Most aortic aneurysms, about 75%, arise in the section running through one's abdomen and are thus referred to as "abdominal aneurysms". Other aortic aneurysms, referred to as "thoracic aneurysms", occur in the section of the aorta running through one's chest.

One cause of aortic aneurysms, in particular abdominal aortic aneurysms, is the degradation of elastin and collagen in the aortic wall, leading to dilatation, progressive growth, and eventual rupture. The rupturing of an aortic aneurysm causes life-threatening internal bleeding. Of course, the larger an aneurysm is, the higher the risk of it rupturing. Approximately 15,000 people die each year in the United States of a ruptured aortic aneurysm. If detected in time, an aortic aneurysm can usually be repaired by surgery.

Unfortunately, most aortic aneurysms have no symptoms and are only uncovered during an evaluation of another condition, such as on a chest X-ray, echocardiogram, magnetic resonance imaging (MRI) or computerized tomography (CT) scan. The treatment for an aneurysm is dependent upon its size, location and the general health of the patient. If the aneurysm is small and is discovered during a routine physical, i.e. the patient did not have any symptoms leading to its discovery, the treatment of the aneurysm usually involves its periodic evaluation. A yearly ultrasound is most often used to track the growth of the aneurysm. Surgery is generally not recommended for aneurysms smaller than 5 cm in diameter. Aneurysms 5 cm or larger in diameter, aneurysms which are being monitored and have found to be rapidly increasing in size (i.e. more than 1 cm per year) or aneurysms causing symptoms in the patient, usually require surgery to prevent complications such as rupture.

The surgical treatment of an aneurysm typically involves the use of a replacement vessel or an artificial prosthesis following the excision of the aneurysm. In other instances, stress can be relieved in the affected vessel by implanting a supporting structure such as a stent or other intravascular device therein.

Prophylactic methods for preventing the formation of aneurysms have predominantly been directed to reducing the mechanical stress on the vascular tissue by reducing the patient's blood pressure. Unfortunately, the drugs used to reduce blood pressure have also been shown to cause undesirable side effects over long-term use, do not improve the structure of the effected blood vessel and have no success in regressing the growth of already-existing aneurysms.

As mentioned above, it has been determined that one specific cause of some aneurysms is the degradation of elastin and collagen in the aortic wall. A family of enzymes, known as the matrix metalloproteinases (MMPs), is believed to be responsible for triggering and controlling the rate of the degradation process described above (see US-5,834,449 to Thompson et al.). It has been shown that inhibitors of MMPs can slow down, and in some cases, arrest the degradation process. One example of such an inhibitor, doxycycline, has been shown to be effective at slowing the progression of aneurysms in animal models (see Kaito, K. et al., "Doxycycline Treatment In A Model Of Early AAA," Surgery Today, 33:426-433 (2003) and Petrinic, D. et al., "Doxycycline Inhibition Of Aneurismal Degeneration In An Elastase-Induced Rat Model Of AAA: Preservation Of Aortic Elastin Associated With Suppressed Production Of 92kD Gelatinase," J. Vascular Surgery, Vol. 23, No. 2, pp.336-346 (1996)).

The delivery of the MMP inhibitors to the aneurismal site is very important to the effective treatment of the degradation disease. As disclosed in US-5,834,449, delivery of the inhibitor may be accomplished by incorporating the inhibitor compound into an implantable device, such as stents or grafts, catheters, embolic coils, filters, cannulas, prostheses and other such devices known in the art. By incorporating the inhibitor compound into a coating on the implantable device or into the implantable device itself where the device is made of a polymeric material, the compound can be delivered *in situ* in a controlled-release fashion. By using such an implantable device, additional direct structural support for the involved vessel is provided. Although providing some function, the implantable device remains in the patient.

The present invention is directed to the treatment of aneurysms in a mammal, particularly aortic aneurysms, and most specifically small abdominal aortic aneurysms, by the implantation of a polymeric, biodegradable device incorporating an anti-aneurismal effective amount of a MMP inhibitor. The present invention is further directed to the polymeric, biodegradable implantable device produced from a polymeric material subject to biological degradation in the presence of organic liquids and having an anti-aneurismal effective amount of a MMP inhibitor incorporated therein. Not only can the treatment in accordance with the present invention prevent the inception and growth of aneurysms, it can also induce regression of established aneurysms. Once the device is delivered to or near the aneurysm site and the MMP inhibitor has been delivered, the implanted device biodegrades and eventually no longer exists. As such, a second surgical procedure is not needed to remove the drug-delivering device.

The device of the present invention can be used in a method for treating an aortic aneurysm in a mammal comprising delivering a polymeric, biodegradable implantable device incorporating at least one drug to an aneurismal site in said mammal, said at least one drug comprising an anti-aneurismal effective amount of a MMP inhibitor.

The aortic aneurysm may be an abdominal aortic aneurysm. The aneurysm may have a a diameter of 5 cm or less. The mammal may be a human.

The polymeric, biodegradable implantable device is preferably a stent, graft, catheter, embolic coil, filter, cannula, prosthesis or the like.

The polymeric, biodegradable implantable device preferably comprises a biodegradable polymeric material which is subject to biological degradation in the presence of organic liquids.

The biodegradable polymeric material is preferably selected from the group consisting of materials made of lactic and glycolic acid, a polyester composed of homopolymers or copolymers of glycolide and lactide, poly(dioxanone), poly(trimethylene carbonate) copolymers, poly (ε-caprolactone) homopolymers and copolymers, polyanhydrides, polyorthoesters and polyphosphazenes.

The biodegradable polymeric material is preferably a blend of polylactide and trimethylene carbonate.

The MMP inhibitor is preferably a tetracycline compound. The tetracycline compound may be doxycycline, aureomycin or chloromycin. The tetracycline compound may also be a chemically-modified tetracycline.

The anti-aneurismal amount of the MMP inhibitor is preferably between about 0.1 mg/kg/day to about 30 mg/kg/day, more preferably between about 1 mg/kg/day and about 18 mg/kg/day.

The MMP inhibitor may be directly incorporated into the polymeric, biodegradable implantable device. The MMP inhibitor may also be incorporated into a coating which is applied to the polymeric, biodegradable implantable device.

Delivering of the polymeric, biodegradable implantable device incorporating at least one drug to an aneurismal site in the mammal preferably may comprise delivery of the device directly to the aneurismal site or delivery of the device upstream of the aneurismal site.

The present invention is directed to a drug-device combination product and method for the treatment of small aneurysms, specifically abdominal aortic aneurysms, in a mammal. In accordance with the present invention, one or more drugs are incorporated into an implantable polymeric, biodegradable device via a coating or by direct incorporation into the device material. With the use of the method and implantable device according to the present invention, local delivery of the device allows the drug or drugs to be delivered to the disease site directly, allows two or more drugs to be delivered simultaneously leading to a more effective treatment, minimizes any side-effects from the drug or drugs in other parts of the body, provides additional structural support while the structure is still intact and the disease site is being treated, and provides for the "removal" of the device after treatment as the device degrades after its usefulness. That is, a second surgical; intervention is not required for the removal of the device.

More specifically, the present invention is directed to a method for treating aortic aneurysms in a mammal comprising delivering a polymeric, biodegradable implantable device incorporating at least one drug to or near an aneurismal site in said mammal, said at least one drug comprising an anti-aneurismal effective amount of a MMP inhibitor. By the phrase "treating aortic aneurysms", it is meant to cover both the treatment of already-existing aortic aneurysms in hopes of inducing the regression of its growth, as well as the prevention of aneurysms altogether. Although the present invention can be used for the purposes of treating all types of aneurysms, it is especially directed to the treatment of aortic aneurysms, and most specifically directed to the treatment of abdominal aortic aneurysms.

Due to the fact that most aortic aneurysms having a diameter of 5 cm or greater require surgery, the present invention is mostly directed to the treatment of aneurysms having a diameter of 5 cm or less.

As already indicated, the present invention is directed to the treatment of aneurysms in mammals, of which humans are the most important. The present invention can also be used for the treatment of other mammals, such as dogs, cats, horses and cows.

In accordance with the present invention, a polymeric, biodegradable device is implanted in the mammal at or near the aneurismal site. That is, as an alternative to implanting the polymeric, biodegradable device directly at the aneurismal site, the device can also be implanted in the part of the vessel that is upstream of the diseased aorta. Under such a scenario, the drug or drugs would then be delivered downstream to treat the diseased site. One challenge of the current grafts being used for treating AAA is the migration of the device over time especially because of the anatomy of the abdominal aorta. A biodegradable device such as a stent, implanted at a region upstream, may be easier to anchor and at the same time, the drug or drugs can be carried to the diseased site by normal blood flow. Said implantable polymeric, biodegradable device can be any intravascularly implantable device known in the art made of a suitable polymeric, biodegradable material. Examples of implantable devices which can be used in accordance with the present invention are stents or grafts, catheters, embolic coils, filters, cannulas, prostheses and other such devices known in the art. Any of these devices allow for the intravascular delivery of the drug or drugs to the aneurismal site. The use of a biodegradable stent or graft is preferred.

The implantable intravascular device to be used in accordance with the present invention is made of a polymeric, biodegradable material. Any suitable polymeric, biodegradable material known in the art may be used. The general criteria for selecting a polymer for use as a biomaterial is to match the mechanical properties and the time of degradation to the needs of the application. These criteria are well-known in the art. For example, the ideal polymer for a particular application would be configured so that: (a) its mechanical properties match the application, i.e. remain sufficiently strong until the surrounding tissue has been treated or healed; (b) does not result in a toxic or inflammatory response; (c) is easily sterilized; (d) is easily made into the final product form; (e) has an acceptable shelf-life; and (f) is metabolized in the body after fulfilling its purpose, i.e. does not leave any trace.

As alluded to above, an implantable device prepared from a biodegradable polymer has the advantage in that it can be engineered to degrade at a particular desired rate and act as the basis for drug delivery, either as a drug delivery system alone or in conjunction to functioning as a medical device. Factors affecting the mechanical performance of biodegradable polymers are well known to the polymer scientist, and include monomer selection, initiator selection, process conditions, and the presence of additives. These factors ultimately affect the polymer's hydrophilicity, crystallinity, melt and glass-transition temperatures, molecular weight, molecular-weight distribution, end groups, sequence distribution (random versus blocky), and presence of residual monomer or additives. In addition, the polymer scientist working with biodegradable materials must evaluate each of these variables for its effect on biodegradation.

Examples of well-known polymeric, biodegradable materials include materials based on lactic and glycolic acid, poly(dioxanone), poly(trimethylene carbonate) copolymers, and poly (ε-caprolactone) homopolymers and copolymers. In addition to these approved materials which may be used for implantable devices, much ongoing research is directed to polyanhydrides, polyorthoesters, polyphosphazenes, and other biodegradable polymers. The most preferred devices to be used in accordance with the present invention are comprised of polyesters composed of homopolymers or copolymers of glycolide and lactide. Other preferred materials are made from copolymers of trimethylene carbonate and ε-caprolactone. One specific example of a biodegradable intravascular stent is one molded from a blend of polylactide and trimethylene carbonate.

As indicated above, in accordance with the present invention, at least one drug is incorporated into the polymeric, biodegradable implantable device, said drug comprising an anti-aneurismal effective amount of a MMP inhibitor. Numerous MMP inhibitors are already known in the art suitable for use in inhibiting the growth and establishment of aneurysms. One such known family of inhibitors having an anti-aneurismal effect is tetracycline compounds. Preferred tetracycline compounds include tetracycline and derivatives thereof, such as aureomycin and chloromycin. Another preferred tetracycline compound is doxycycline.

In addition, the MMP inhibitor used in accordance with the present invention may comprise a chemically-modified tetracycline (CMT). Such CMTs are known in the art and include, but are not limited to, 4-dedimethylaminotetracycline (CMT-1), 4-dedimethylamino-5-oxytetracycline, 4-dedimethylamino-7-chlorotetracycline (CMT-4), 4-hydroxy-4-dedimethylaminotetracycline (CMT-6), 5 a,6-anhydro-4-hydroxy-4-dedimethylaminotetracycline, 6-demethyl-6-deoxy-4-dedimethylaminotetracycline (CMT-3), 4-dedimethylamino-12a-deoxytetracycline (CMT-7), 6-α-deoxy-5-hydroxy-4-dedimethylaminotetracycline (CMT-8). 6-α-benzylthiomethylenetetracycline, the mono-N-alkylated amide of tetracycline, 6-fluoro-6-demethyltetracycline, and 11-α-chlorotetracycline.. Other suitable inhibitors used in accordance with the present invention are any effective inhibitors of MMP-related proteolytic activity. This includes inhibitors of the synthesis or expression of involved MMPs, as well as inhibitors of the proteolytic activity of an expressed MMP. One or more combinations of the above can also be used.

The anti-aneurismal effective amount of the MMP inhibitor to be used is such that the skilled artisan can determine an appropriate amount, i.e. dosages which are effective to achieve the desired result. More particularly, it is desired to employ a maximum dosage that is effective for treating the aneurismal site while at the same time not causing any undesirable side effects. For example, administration of a tetracycline compound in a dosage of more than about 50 mg/kg/day would probably result in unwanted side effects. Thus, it is recognized in the art that a dosage of a tetracycline compound between an amount of about 0.1 mg/kg/day to about 30 mg/kg/day is more acceptable, with a preferred dosage being between about 1 mg/kg/day and about 18 mg/kg/day.

Depending upon the condition of the mammal being treated, one or more drugs can be incorporated into the biodegradable device. Other drugs incorporated into the biodegradable device may have the same target but may fight the target via a different mechanism, or have a different target altogether. Said one or more drugs, for example, in addition to the MMP inhibitor, could be either a therapeutic or diagnostic agent. Examples of therapeutic agents include proteins (e.g., insulin and other hormones), polysaccharides (e.g., heparin), anaesthetics, antibiotics and chemotherapeuric agents. Examples of diagnostic agents include imaging and contrast agents.

The delivery of the MMP inhibitor (and any other drugs incorporated therein) to the aneurismal site is achieved in accordance with the present invention by incorporating the inhibitor compound into the polymeric, biodegradable implantable device. This can be done by either applying a coating containing the inhibitor compound to the implantable device or by incorporating the inhibitor compound directly into the polymeric, biodegradable device. In both cases, the compound will be delivered in a controlled-release fashion directly at or near the aneurismal site.

Time-release or controlled-delivery of the MMP inhibitor compound incorporated into the polymeric, biodegradable implantable device of the present invention is employed in accordance with known methods in the art.

## Claims

1. A polymeric, biodegradable implantable device comprising a polymeric, biodegradable material and an anti-aneurismal amount of a MMP inhibitor incorporated therein.

2. The polymeric, biodegradable implantable device according to claim 1, wherein the polymeric, biodegradable material is selected from the group consisting of material made of lactic and glycolic acid, poly(dioxanone), poly(trimethylene carbonate) copolymers, poly (ε-caprolactone) homopolymers and copolymers, polyanhydrides, polyorthoesters and polyphosphazenes.

3. The polymeric, biodegradable implantable device according to claim 1, wherein the biodegradable polymeric material is a blend of polylactide and trimethylene carbonate.

4. The polymeric, biodegradable implantable device according to claim 1, wherein the MMP inhibitor is a tetracycline compound.

5. The polymeric, biodegradable implantable device according to claim 4, wherein the tetracycline compound is doxycycline, aureomycin or chloromycin.

6. The polymeric, biodegradable implantable device according to claim 4, wherein the tetracycline compound is a chemically-modified tetracycline.

7. The polymeric, biodegradable implantable device according to claim 1, wherein the anti-aneurismal amount of the MMP inhibitor is between about 0.1 mg/kg/day to about 30 mg/kg/day.

8. The polymeric, biodegradable implantable device according to claim 1, wherein the MMP inhibitor is directly incorporated into the polymeric, biodegradable material.

9. The polymeric, biodegradable implantable device according to claim 1, wherein the MMP inhibitor is incorporated into a coating which is applied to the polymeric, biodegradable implantable device.
